(19) Europäisches Patentamt / European Patent Office / Office européen des brevets

(11) **EP 3 182 118 B1**

(12) **EUROPÄISCHE PATENTSCHRIFT**

(45) Veröffentlichungstag und Bekanntmachung des Hinweises auf die Patenterteilung:
**06.03.2019 Patentblatt 2019/10**

(51) Int Cl.:
***G01N 33/22*** *(2006.01)*     ***G01N 33/00*** *(2006.01)*

(21) Anmeldenummer: **15003639.0**

(22) Anmeldetag: **19.12.2015**

(54) **VERFAHREN UND MESSVORRICHTUNG ZUR BESTIMMUNG VON GASEIGENSCHAFTEN MITTELS KORRELATION**

METHOD AND MEASURING DEVICE FOR DETERMINING GAS PROPERTIES USING CORRELATION

PROCEDE ET DISPOSITIF DE MESURE POUR LA DETERMINATION DE PROPRIETES DE GAZ A L'AIDE DE CORRELATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.06.2017 Patentblatt 2017/25**

(73) Patentinhaber: **MEMS AG
5200 BRUGG (CH)**

(72) Erfinder:
• **PRÊTRE, Philippe
5405 Dättwil (CH)**
• **KEMPE, Andreas
8049 Zürich (CH)**

(74) Vertreter: **Steiner, Peter
Patente + Marken
Untere Wolfackerstrasse 16
8280 Kreuzlingen (CH)**

(56) Entgegenhaltungen:
EP-A1- 0 715 169     EP-A1- 2 015 056
EP-A1- 2 806 271     EP-A2- 0 939 317
DE-A1- 19 921 167

**Beschreibung**

Die Erfindung bezieht sich auf ein Verfahren und auf eine Messvorrichtung zur Bestimmung von Gaseigenschaften mittels Korrelation.

Die (Erd-)Gaszusammensetzung und damit die Gasbeschaffenheit wird künftig aufgrund von neuen Herkunftsquellen (Biogas, verflüssigtes Erdgas aus allen Weltgegenden, Wasserstoff aus der Verwertung von überschüssigem Strom bei der alternativen Energiegewinnung) mehr und häufiger schwanken als heute und damit in Gasanwendungsprozessen unterschiedliche, mitunter auch negative Auswirkungen haben. Mit Kenntnis der relevanten Gaseigenschaften vor Ort könnten die Prozesse auf die wechselnde Gasqualität eingestellt werden, um einen optimalen und sicheren Betrieb zu gewährleisten. Zu den in diesem Zusammenhang relevanten Gaseigenschaften gehören zum Beispiel der Wobbe Index für Brennersteuerungen, die Luftzahl bei Energiegewinnungsanlagen, wie beispielsweise Industrieöfen oder Brennstoffzellen, die Methanzahl für Gasmotoren oder der Brennwert zur Abrechnung der bezogenen Energiemenge. Meist sind diese Gaseigenschaften nur mit grossem Aufwand direkt messbar, sodass eine Vor-Ort-Bestimmung in der Regel nicht wirtschaftlich ist.

In EP 2 015 056 A1 wird ein thermischer Durchflusssensor zur Bestimmung einer brenntechnisch relevanten Grösse beschrieben, basierend auf einer Wärmeleitfähigkeitsmessung bei quasi bekanntem Massenfluss. Hierzu wird eine kritische Düse benutzt, um den Massenfluss konstant zu halten.

In EP 0 715 169 A1 wird ein Verfahren und eine Vorrichtung beschrieben, in welchen die Dichte und/oder Viskosität eines Brenngases gemessen wird, um aus den Messwerten den Brennwert oder Wobbeindex zu bestimmen.

In dem in DE 199 21 167 A1 beschriebenen Verfahren wird die Schallgeschwindigkeit oder Dichte sowie die Infrarotabsorption eines Brenngases ermittelt, um daraus mittels Korrelation den Brennwert oder Wobbeindex zu bestimmen.

In dem aus EP 0 939 317 A2 bekannten Verfahren wird aus dem Brennwert oder der Dielektrizitätskonstante einerseits und der Dichte oder Schallgeschwindigkeit andererseits die Gaszusammensetzung bestimmt.

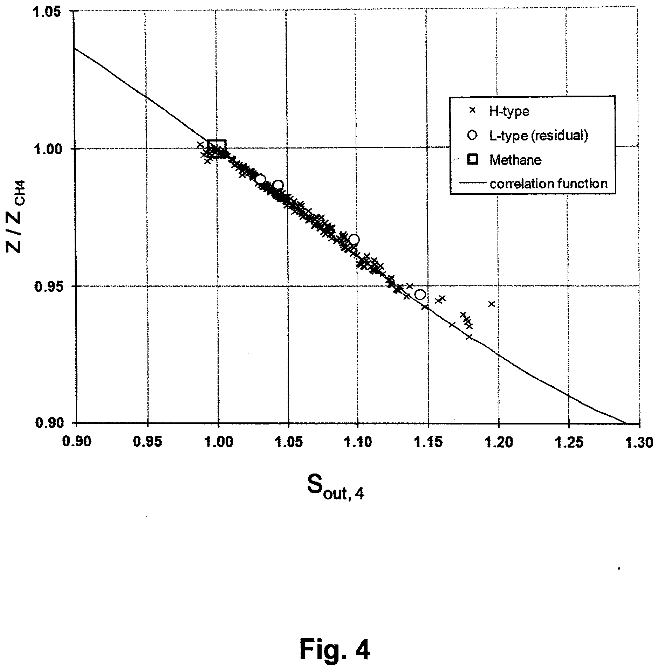

Aus EP 2 806 271 A1 ist ein Verfahren sowie eine Messvorrichtung zur Bestimmung von physikalischen Gaseigenschaften bekannt, in welchem aus mehreren, einfach zu bestimmenden physikalischen Grundgrössen eines Gases, wie z.B. der Wärmeleitfähigkeit, mittels Korrelation eine gesuchte Gaseigenschaft ermittelt wird. Mit dieser einfachen Messvorrichtung wird dann auch eine Vor-Ort-Bestimmung der gesuchten Gaseigenschaft ermöglicht. Zwar wird in Gleichung 16 der erwähnten Anmeldung eine mögliche Korrelationsfunktion genannt, es wird jedoch nicht ausgeführt, mit welcher Genauigkeit diese Korrelation für die in Fig. 4 gezeigten Gasgemische gilt.

Abhängig von der Gaseigenschaft und der Auswahl von Gasgemischen, für welche die Gaseigenschaft bestimmt werden soll, kann es schwierig sein, mit nur einer Korrelation die gesuchte Gaseigenschaft mit ausreichender Genauigkeit aus den Messgrössen zu bestimmen.

Zweck der Erfindung ist es deshalb, ein Verfahren und eine Messvorrichtung anzugeben, mit welchem die Korrelation von Gaseigenschaften verbessert werden kann und/oder die Auswahl an Gasgemischen, für welche die Korrelation eine gewünschte Genauigkeit aufweist, zu vergrössem.

Diese Aufgabe wird durch ein Verfahren nach Anspruch 1 und durch eine Messvorrichtung nach Anspruch 14 gelöst.

Das vorliegende Verfahren geht von der Beobachtung der Anmelderin aus, dass es innerhalb einer Menge von Gasgemischen, für welche eine Gaseigenschaft bestimmt werden soll, Gruppen gibt, in denen die Gasgemische ein physikalisch ähnliches Verhalten aufweisen. Im Zusammenhang mit der Bestimmung von Gaseigenschaften bedeutet das, dass die Korrelation zwischen den Messgrössen und der gesuchten Gaseigenschaft innerhalb einer solchen Gasgemischgruppe besser ist als in der gesamten Menge, und dass sich die Gasgemische einer solchen Gruppe mittels physikalischer Messgrössen, welche im Zuge des Verfahrens erfasst werden, von den übrigen Gasgemischen der Menge separieren lassen.

Es liegt nahe, das physikalisch ähnliche Verhalten der Gasgemische einer Gasgemischgruppe mit der Gleichartigkeit der Zusammensetzung der Gasgemische in Verbindung zu bringen. So bilden beispielsweise die Gasgemische vom Typ $CH_4 + H_2$ aus einem der nachstehend beschriebenen Ausführungsbeispiele eine Gasgemischgruppe. Die Gleichartigkeit der Zusammensetzung ist jedoch keine Bedingung für das vorliegende Verfahren. Wichtig ist in diesem Zusammenhang lediglich, dass die Korrelation innerhalb einer Gasgemischgruppe besser ist als in der gesamten Menge, und dass sich die Gasgemische einer Gasgemischgruppe von den übrigen Gasgemischen der Menge separieren lassen.

Im nachfolgend vorgestellten Verfahren wird die Korrelation durch eine Korrelationsfunktion $f_{corr}$ für die Gaseigenschaft Q beschrieben, wobei $f_{corr}$ eine Funktion des "sensor output" $S_{out}$ ist

$$Q = f_{corr}(sensor\ output) := f_{corr}(S_{out}). \qquad (1)$$

Dem Verfahren liegt ein schritt- bzw. abschnittweises Vorgehen zugrunde, in dem versucht wird, den Sensor-Output $S_{out,1}$ in einem ersten Schritt derart zu definieren, dass eine erste Gruppe von Gasgemischen aus einer Menge sich entlang der $S_{out,1}$-Achse von allen anderen Gasgemischen der Menge separieren und mit einer ersten Korrelationsfunktion $f_{corr,1}$ korrelieren lässt, um so im nächsten Schritt nicht mehr betrachtet werden zu müssen. Die restlichen oder übrigen Gasgemische der Menge werden dann in einem zweiten Schritt mit einem zweiten, neu zu definierenden Sensor-Output $S_{out,2}$ verglichen, um eine zweite Gasgemischgruppe mit einer zweiten Korrelationsfunktion $f_{corr,2}$ separat korrelieren zu können. Das Vorgehen kann beliebig oft wiederholt werden, bis der gewünschte Grad an Korrelationsgüte erreicht ist.

Der i-te Sensor-Output $S_{out,i}$ (i=1,...,n) ist dabei eine Funktion $f_i$ von einer oder mehreren physikalischen Messgrössen $\mu_j$ (j=1,...,m) eines oder mehrerer Sensoren:

$$S_{out,i} = f_i(\mu_1, \ldots, \mu_m). \qquad (2)$$

Die Korrelationsfunktionen $f_{corr,i}$ (i=1...n) kann, bezogen auf $S_{out,i}$, abschnittsweise eine andere sein, ja, sogar eine unstetige Änderung von $f_{corr,i}$ an den Abschnittsgrenzen ist möglich.

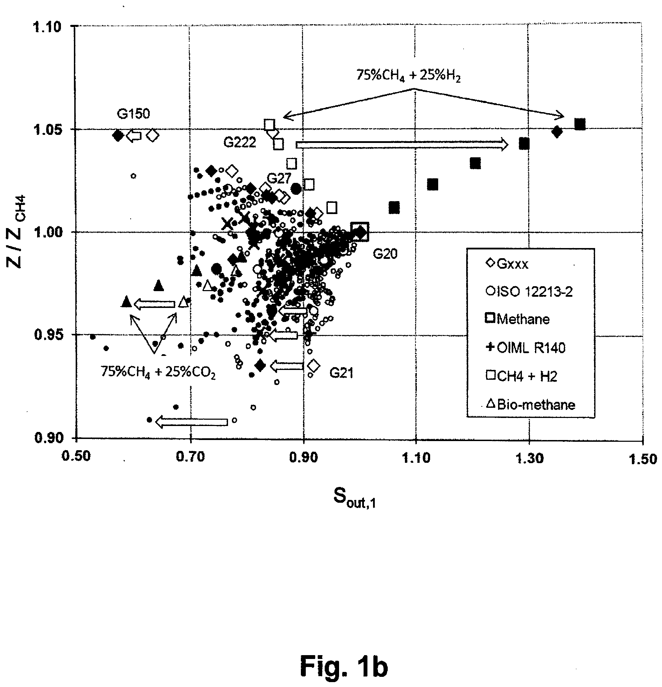

Typisch an diesem Verfahren ist, dass es durch die Einführung des Sensor-Outputs $S_{out,i}$, der eine Funktion physikalischer Messgrössen ist, möglich wird, anhand von tabellierten Werten dieser physikalischen Messgrössen für Gruppen von Gasgemischen, insbesondere gegebene Gasgemischgruppen, eine gezielte Wahl für die Funktionen $f_i$ zu treffen, die dann, wie oben beschrieben, zur Gasgemischgruppenseparation führt. Wird nämlich die Gaseigenschaft Q auf der y-Achse gegen $S_{out,i}$ auf der x-Achse aufgetragen, verschieben sich die Punkte der Gasgemische bei sich ändernder Funktion $f_i$ ausschliesslich parallel zur x-Achse, was das visuelle Mitverfolgen der Auswirkungen der Änderung der Funktion $f_i$ erheblich erleichtert (siehe Fig. 1b).

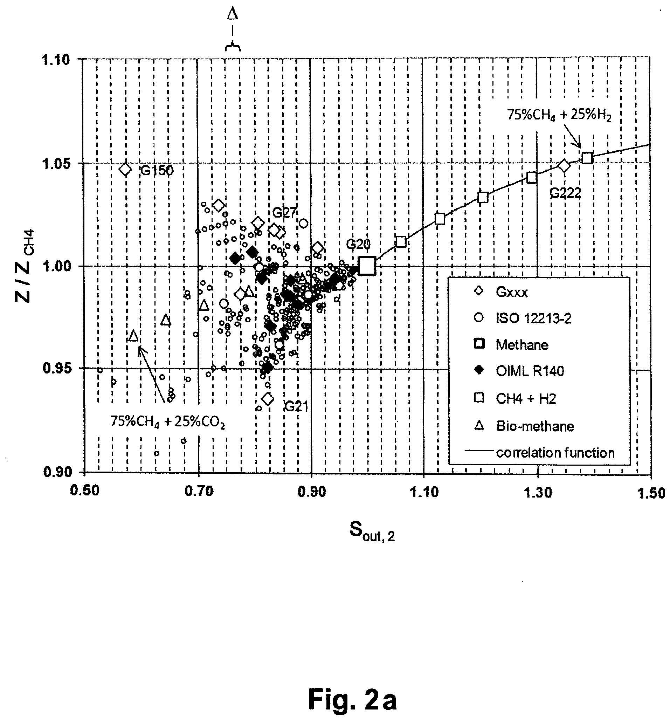

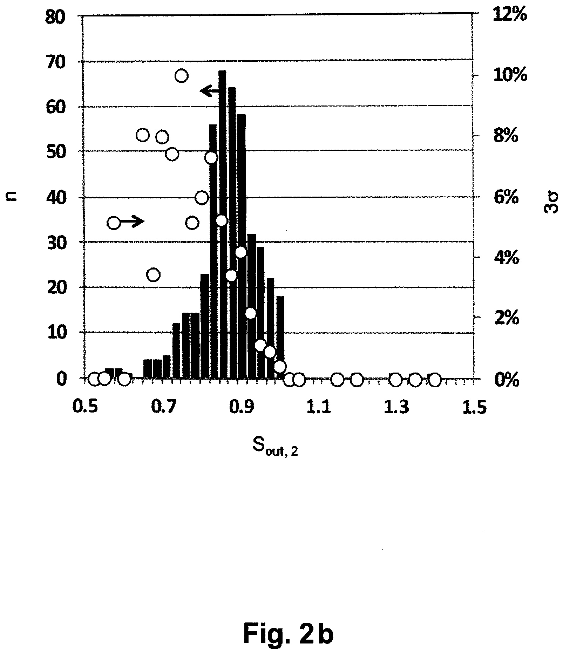

Das visuelle Mitverfolgen lässt sich auch mit einem Computerprogramm wie folgt automatisieren: die Funktionsparameter $p_{fi}$ von $f_i$, wie beispielsweise Polynomkoeffizienten, Exponenten oder Konstanten, werden für jede Funktion $f_i$ in einer Menge von möglichen Sensor-Output-Funktionen innerhalb von einzugebenden Grenzwerten für $p_{fi}$ variiert, z.B. mittels eines Monte-Carlo-Auswahlverfahrens. Gleichzeitig wird die Anzahl Doppeldeutigkeiten gezählt, d.h. die Anzahl Ereignisse, für die zwei oder mehrere Gasgemische bei "gleichem" Sensor-Output $S_{out,i}$ "unterschiedliche" Werte für die zu korrelierende Grösse Q zeigen. "Gleich" und "unterschiedlich" können dabei auch inner- bzw. ausserhalb vorzugebender Werteintervalle bedeuten. In Fig. 2a ist beispielsweise ein möglicher Wert für die Intervallbreite $\Delta$ des Sensor-Output $S_{out,2}$ eingezeichnet. Ziel ist es z.B., diejenige Funktion $f_i$ und denjenigen Funktionsparametersatz $p_{fi}$ zu bestimmen, bei denen am wenigsten solche Doppeldeutigkeitsereignisse auftreten. Wird entlang der x-Achse ($S_{out,i}$) ein Histogramm dieser Ereignisse erstellt, können, wie in Fig. 2b gezeigt, diejenigen Abschnitte entlang der x-Achse ermittelt werden, wo und für welche Gasgemischgruppen eine gute bzw. bessere Korrelation möglich ist (mit einer minimalen Anzahl n solcher Doppeldeutigkeitsereignisse bzw. minimalen Streuung $3\sigma$ der Q-Werte bei Doppeldeutigkeitsereignissen in einem Intervall). An Stelle der Minimumsuche kann für das Auswahlverfahren beispielsweise auch die maximal erlaubte Anzahl n der Doppeldeutigkeitsereignisse bzw. die maximal erlaubte Streuung $3\sigma$ der Q-Werte bei Doppeldeutigkeitsereignissen in einem Intervall vorgegeben werden.

Implizit ist mit dem vorliegenden Verfahren zur Bestimmung von Gaseigenschaften auch eine physikalische Definition einer Gasgemischgruppe gegeben: Gasgemische, die physikalisch ähnlich sind, lassen sich von Gasgemischen, die zwar untereinander ebenfalls physikalisch ähnlich sein können, sich jedoch in einem physikalischen Parameter der Ähnlichkeit von der ersten Gasgemischgruppe unterscheiden, separieren.

Als Beispiel für einen physikalischen Parameter der Ähnlichkeit seien die thermischen Freiheitsgrade von Gasmolekülen genannt. Gemäss dem Äquipartitionstheorem entfällt im thermischen Gleichgewicht auf jeden Freiheitsgrad die gleiche mittlere Energie 1/2kT. Brennbare Gase wie Methan, Ethan und alle weiteren höheren Kohlenwasserstoffe verhalten sich wie höheratomige Gase mit 6 Freiheitsgraden, wohingegen inerte Gasbestandteile wie Stickstoff, Sauerstoff und Argon eher einatomigen Gasen mit 5 Freiheitsgraden zuzuordnen sind, da gewisse Freiheitsgrade bei Raumtemperatur noch "eingefroren" sind. Diese Tatsache wirkt sich auf die Wärmekapazität dieser Moleküle aus (mehr Freiheitsgrade = höhere Wärmekapazität). Gleichzeitig, diesmal allerdings chemisch bedingt, tragen die inerten Moleküle nichts zum Brennwert (Q) bei. In diesem Fall kann also über die Messung der Wärmekapazität $c_p = \mu$ die Gasgruppe mit hohem Brennwert (H-Gase) von derjenigen mit niedrigem Brennwert (L-Gase) separiert werden.

Weitere Beispiele für physikalische Messgrössen $\mu_i$ sind die Dichte p, die Wärmeleitfähigkeit $\lambda$, die Schallgeschwindigkeit $c_s$, die Dielektrizitätskonstante $\varepsilon$ u.a..

Beispiele für die zu bestimmende Gaseigenschaft Q sind z.B. die Viskosität (wichtig bei der Auslegung von Rohrleitungssystemen), der Realgasfaktor Z (wichtig für Abrechnungszwecke bei Gastransportleitungen), aber auch die Flammgeschwindigkeit (bei Wärmeanwendungen in der Prozessindustrie) oder direkte Prozessparameter wie etwa der Zündwinkel bei Gasmotoren. Letztere sind deshalb von speziellem Interesse, weil in diesem Fall die physikalischen Parameter der Ähnlichkeit anhand der Gaszusammensetzung schwierig zu erfassen sind, da Gaseigenschaften im "traditionellen" Sinn gas-, Prozessparameter hingegen prozessgegeben sind.

In dem Verfahren zur Bestimmung von Gaseigenschaften gemäss vorliegender Erfindung wird eine zu bestimmende Gaseigenschaft, beispielsweise eine physikalische Gaseigenschaft, aus physikalischen Messgrössen der Gase und/oder Gasgemische korreliert, wobei die physikalischen Messgrössen mittels einer Sensor-Outputfunktion zu einem Sensor-Output zusammengefasst werden, und der Sensor-Output mit einem Grenzwert $S_{out}^{sep}$ verglichen wird, um festzustellen, ob der Sensor-Output innerhalb der Menge G von Gasen und/oder Gasgemischen, auf die das Verfahren Anwendung findet, zu einer Gruppe von Gasen und/oder Gasgemischen, im Folgenden Gasgemischgruppe GG genannt, gehört, in welcher die Korrelation zwischen dem Sensor-Output und der zu bestimmenden Gaseigenschaft besser ist als in der gesamten Menge G. Wenn der Sensor-Output zur genannten Gasgemischgruppe GG gehört, wird die Gaseigenschaft mittels einer Korrelationsfunktion, die für die Gasgemischgruppe spezifisch ist, aus dem Sensor-Output korreliert.

In einer vorteilhaften Ausführungsform des Verfahrens wird die Zugehörigkeit zu einer Gasgemischgruppe in zwei, drei, vier oder mehr Schritten geprüft, indem jeweils die physikalischen Messgrössen mittels einer Sensor-Outputfunktion, die für die nach Separierung der vorangehenden Gasgemischgruppe oder -gruppen übrig gebliebenen Gase und/oder Gasgemische $G_{rest,i}$ der Menge, im Folgenden übrig gebliebene Gase und/oder Gasgemische $G_{rest,i}$ genannt, spezifisch ist, zu einem weiteren Sensor-Output zusammengefasst werden, und der weitere Sensor-Output mit einem weiteren Grenzwert $S_{out,i}^{sep}$ verglichen wird, um festzustellen, ob der weitere Sensor-Output innerhalb der übrig gebliebenen Gase und/oder Gasgemische $G_{rest,i}$ zu einer weiteren Gasgemischgruppe $GG_i$ gehört, in welcher die Korrelation zwischen dem weiteren Sensor-Output und der zu bestimmenden Gaseigenschaft besser ist als innerhalb der übrig gebliebenen Gase und/oder Gasgemische $G_{rest,i}$. Wenn der weitere Sensor-Output zur genannten weiteren Gasgemischgruppe $GG_i$ gehört, wird die Gaseigenschaft mittels einer für die weitere Gasgemischgruppe spezifischen Korrelationsfunktion aus dem weiteren Sensor-Output korreliert.

Wenn der Sensor-Output nicht zu einer der genannten Gasgemischgruppen gehört, kann die Gaseigenschaft mittels einer Korrelationsfunktion, die für die übriggebliebenen Gase und/oder Gasgemische spezifisch ist, mit dem Sensor-Output korreliert werden.

In einigen Fällen kann es vorteilhaft sein, den Sensor-Output vor der Korrelation mit einer weiteren Sensor-Outputfunktion zu verändern, um die Korrelation zwischen dem Sensor-Output und der Gaseigenschaft vorzubereiten oder zu verbessern und/oder die Suche einer geeigneten Korrelationsfunktion zu vereinfachen.

In einer weiteren vorteilhaften Ausführungsform des Verfahrens werden die Sensor-Outputfunktion und ein Grenzwert $S_{out}^{sep}$ für den Sensor-Output derart festgelegt, dass mittels desselben aus einer Menge G von Gasen und/oder Gasgemischen, für welche die Gaseigenschaft bestimmt wird, eine Gasgemischgruppe GG separiert wird, innerhalb welcher die Korrelation zwischen dem Sensor-Output und der gesuchten Gaseigenschaft besser ist als in der gesamten Menge G.

Die Separation von Gasgemischgruppen kann auch in zwei, drei, vier oder mehr Schritten erfolgen, indem jeweils aus den übrigen, d.h. aus den nach der vorangehenden Separation übrig gebliebenen Gasen und/oder Gasgemischen $G_{rest,i}$ eine neue Menge gebildet wird, aus der eine weitere Gasgemischgruppe mit einer eigenen Sensor-Outputfunktion und mit einem eigenen Grenzwert $S_{out,i}^{sep}$ für den Sensor-Output separiert wird, und indem die Korrelation aus dem Sensor-Output für die Gase und/oder Gasgemische der weiteren Gasgemischgruppe mit einer eigenen Korrelationsfunktion erfolgt.

Typisch gelten für die Gase und/oder Gasgemische der Gasgemischgruppe GG die Beziehung $S_{out}^{GG} < S_{out}^{sep}$ und für die übrigen Gase und/oder Gasgemische $G_{rest}$ der Menge die Beziehung $S_{out}^{Grest} \geq S_{out}^{sep}$, bzw. fallweise, an Stelle der genannten Beziehungen, die Beziehungen $S_{out}^{GG} > S_{out}^{sep}$ und $S_{out}^{Grest} \leq S_{out}^{sep}$.

Unabhängig von den oben beschriebenen Ausführungsformen und -varianten kann das Verfahren automatisch ausgeführt werden, zum Beispiel in einer Messvorrichtung.

Mit Vorteil werden die Sensor-Outputfunktion oder Sensor-Outputfunktionen und/oder der oder die Grenzwerte $S_{out}^{sep}$, $S_{out,i}^{sep}$ für den Sensor-Output und/oder die Korrelationsfunktionen vorab, d.h. typisch vor der Bestimmung von Gaseigenschaften vor Ort, festgelegt, zum Beispiel anhand von Werten der physikalischen Messgrössen und der zu bestimmenden Gaseigenschaft aus Tabellenwerken und/oder Fachliteratur und/oder Datenbanken und/oder Messungen, und bei Bedarf gespeichert.

In einer weiteren vorteilhaften Ausführungsform des Verfahrens werden die Sensor-Outputfunktion oder Sensor-Outputfunktionen und/oder der oder die Grenzwerte $S_{out}^{sep}$, $S_{out,i}^{sep}$ für den Sensor-Output mit einem Computerprogramm ermittelt, indem für jede Funktion $f_i$ in einer Menge möglicher Sensor-Output-Funktionen die jeweiligen Funktionsparameter $p_{fi}$ von $f_i$, wie beispielsweise Polynomkoeffizienten, Exponenten oder Konstanten, innerhalb vorgegebener Grenzwerte für $p_{fi}$ variiert werden, z.B. mittels eines Monte-Carlo-Auswahlverfahrens. Der Sensor-Output-Bereich wird dabei in Intervalle unterteilt und insbesondere in jedem Intervall die Anzahl Doppeldeutigkeiten gezählt, d.h. die Anzahl Ereignisse, für die zwei oder mehrere Gasgemische unterschiedliche Werte für die zu korrelierende Grösse Q zeigen oder die Werte für die zu korrelierende Grösse Q ausserhalb eines vorgegebenen Werteintervalls liegen. Ziel ist es, diejenige Funktion $f_i$ und denjenigen Funktionsparametersatz $p_{fi}$ zu bestimmen, bei welchen am wenigsten solche Doppeldeutigkeitsereignisse auftreten, oder die Streuung $3\sigma$ der Werte für die zu korrelierende Grösse Q bei Doppeldeutigkeitsereignissen in einem Intervall minimal ist, oder eine vorgegebene maximal erlaubte Anzahl $n_{max}$ der Doppeldeutigkeitsereignisse bzw. eine vorgegebene maximal erlaubte Streuung $3\sigma_{max}$ der Q-Werte bei Doppeldeutigkeitsereignissen in einem Intervall nicht überschritten wird.

Zweckmässigerweise wird zudem ermittelt, ab welchem Grenzwert $S_{out,i}^{sep}$ eine je Intervall für die Ermittlung des Grenzwertes vorgegebene Anzahl $\underline{n}_{max}$ der Doppeldeutigkeitsereignisse bzw. eine je Intervall für die Ermittlung des Grenzwertes vorgegebene Streuung $\underline{3\sigma}_{max}$ der Q-Werte bei Doppeldeutigkeitsereignissen nicht überschritten wird.

Typisch sind mindestens zwei oder alle Sensor-Outputfunktionen verschieden voneinander und/oder mindestens zwei oder alle Grenzwerte für den Sensor-Output verschieden voneinander.

Vorteilhafterweise liegen die Punkte der Gase und/oder Gasgemische der Gasgemischgruppe oder -gruppen jeweils auf einer durch eine eindeutige Korrelationsfunktion beschriebenen Linie oder in Toleranzbereichen, die beidseits an eine derartige Linie anschliessen, und die zum Beispiel nicht grösser als 0.25 % oder 0.75 % oder 2 % des Wertes der Gaseigenschaft (Q) sind. Typisch sind mindestens zwei oder alle Korrelationsfunktionen verschieden voneinander.

In einer vorteilhaften Ausführungsvariante sind die Sensor-Outputfunktion oder Sensor-Outputfunktionen vom Typ

$$S_{out,i} = \mu_1^{p_{1,i}} \cdot \ldots \cdot \mu_m^{p_{m,i}}$$

und $p_{1,i}, \ldots, p_{m,i}$ Exponenten, und/oder
die Korrelationsfunktion oder Korrelationsfunktionen vom Typ

$$Q = f_{corr,i}(S_{out,i}) = a_{0,i} + a_{1,i} \cdot S_{out,i} + a_{2,i} \cdot S_{out,i}^2$$

und $a_{0,i}$, $a_{1,i}$ und $a_{2,i}$ Konstanten.

Als Mass für die Genauigkeit der Korrelation kann beispielsweise der Pearson Korrelationskoeffizient verwendet werden, wobei eine bessere Korrelation bedeutet, dass der Pearson Korrelationskoeffizient näher beim Wert +1 oder -1 liegt, und beispielsweise der Absolutwert der Differenz zum Wert +1 bzw. -1 kleiner als 0.3 oder 0.2 oder 0.1 ist.

Der Pearson Korrelationskoeffizient

$$Kor(S_{out}, f_{corr}) = \frac{\sum_{k=1}^{n}(S_{out,k} - \overline{S}_{out})(f_{corr,k} - \bar{f}_{corr})}{\sqrt{\sum_{k=1}^{n}(S_{out,k} - \overline{S}_{out})^2 \cdot (f_{corr,k} - \bar{f}_{corr})^2}} \qquad (3)$$

ist ein Mass für die Abweichung der zwei Variablen $S_{out}$, $f_{corr}$ von der Linearität, wobei $\overline{S}_{out} = \frac{1}{n}\sum_{k=1}^{n}S_{out,k}$ und

$$\bar{f}_{corr} = \frac{1}{n} \sum_{k=1}^{n} f_{corr,k}$$

die Mittelwerte über sämtliche n Gase und Gasgemische sind.

Liegen alle n Punkte der Gase und Gasgemische auf einer Geraden mit positiver Steigung, so hat der Pearson Korrelationskoeffizient den Wert +1. Liegen dagegen alle n Punkte auf einer Geraden mit negativer Steigung, so ist der Wert -1. Sind alle n Punkte stochastisch um einen Punkt verteilt, so besteht keine Korrelation, und der Pearson Korrelationskoeffizient hat den Wert 0.

In der Regel sind die Korrelationsfunktionen, die im oben beschriebenen Verfahren und in den beschriebenen Ausführungsformen und -varianten verwendet werden, nicht linear, d.h., dass die Werte +1 und -1 des Pearson Korrelationskoeffizienten meist nicht erreicht werden. Da die Abweichungen von der Linearität in den meisten Fällen jedoch moderat sind, ist der Pearson Korrelationskoeffizient in der Praxis gut für Vergleichszwecke geeignet, indem die Korrelation oder Korrelationsfunktion zwischen den Variablen $S_{out}$, $f_{corr}$ umso besser oder genauer ist, je näher der Pearson Korrelationskoeffizient beim Wert +1 oder -1 liegt.

Die physikalischen Messgrössen werden vorteilhafterweise mit einem oder mehreren Sensoren erfasst. Als physikalische Messgrössen werden beispielsweise mindestens zwei der folgenden Messgrössen erfasst: Wärmeleitfähigkeit, Wärmekapazität, Wärmediffusivität, Dichte, Strömungsgeschwindigkeit, Massenfluss, Schallgeschwindigkeit, Dielektrizitätskonstante, Viskosität, Infrarot-Absorption, Druck oder Temperatur, wobei die Aufzählung nicht abschliessend ist.

Weiter umfasst die Erfindung eine Messvorrichtung zur Bestimmung von Gaseigenschaften mit einem oder mehreren Sensoren zur Erfassung von physikalischen Messgrössen und mit einer Auswerteeinheit, die zur Ausführung eines Verfahrens gemäss einer oder mehreren der vorangehend beschriebenen Ausführungsformen und -varianten eingerichtet ist.

Die Auswerteeinheit kann beispielsweise zusammen mit dem oder den Sensoren eine Baueinheit bilden, oder die Auswerteeinheit ist in einer separaten oder übergeordneten Recheneinheit ausgebildet.

Das Verfahren und die Messvorrichtung gemäss vorliegender Erfindung zur Bestimmung von Gaseigenschaften haben den Vorteil, dass Dank der Korrelation in mehreren Schritten die Genauigkeit der Bestimmung von Gaseigenschaften aus physikalischen Messwerten der Gase und/oder Gasgemische verbessert werden kann, und dass die Menge der Gase und/oder Gasgemische, für die das Verfahren mit der gewünschten Genauigkeit angewendet werden kann, grösser ist als bei einfachen Korrelationsverfahren. Dank der Korrelation in mehreren Schritten können auch Gasgemische mit Zusammensetzungen, für die eine Bestimmung von Gaseigenschaften bisher nur aufwendig oder nicht mit der nötigen Genauigkeit durchführbar war, in das Verfahren einbezogen werden.

Die Erfindung wird im Folgenden anhand der Zeichnungen näher erläutert. Es zeigen:

Fig. 1a     ein erstes Ausführungsbeispiel mit einer grafischen Darstellung zur Korrelation des Realgasfaktors Z nach einem Verfahren gemäss vorliegender Erfindung,

Fig. 1b     ein Beispiel für die gegenseitige, horizontale Verschiebung der Punkte der einzelnen Gasgemische infolge Veränderung der Sensor-Outputfunktion im ersten Ausführungsbeispiel,

Fig. 2a     ein Beispiel für die Wahl der Sensor-Outputfunktion im ersten Ausführungsbeispiel zur Separierung wasserstoffreicher Gase (sog. "Hythanes"),

Fig. 2b     ein Beispiel eines Doppeldeutigkeitshistogramms und des zu erwartenden Korrelationsfehlers für ein computerbasiertes Auswahlverfahren zur Ermittlung der Sensor-Outputfunktion in dem in Fig. 2a gezeigten Ausführungsbeispiel,

Fig. 3     ein Beispiel für die Wahl der Sensor-Outputfunktion im ersten Ausführungsbeispiel zur Separierung von H- und L-Gasen,

Fig. 4     ein Beispiel für die Wahl der Sensor-Outputfunktion im ersten Ausführungsbeispiel zur Korrelation des Realgasfaktors innerhalb der H-Gase,

Fig. 5     ein Beispiel für die Wahl der Sensor-Outputfunktion im ersten Ausführungsbeispiel zur Korrelation des Realgasfaktors innerhalb der L-Gase,

Fig. 6     ein zweites Ausführungsbeispiel mit einer grafischen Darstellung zur Korrelation der Prandtl-Zahl nach einem Verfahren gemäss vorliegender Erfindung,

Fig. 7    ein Beispiel für die Wahl der Sensor-Outputfunktion im zweiten Ausführungsbeispiel zur Separierung der H-Gase,

Fig. 8    ein Beispiel für die Wahl der Sensor-Outputfunktion im zweiten Ausführungsbeispiel zur Vorbereitung der Korrelation der Prandtl-Zahl innerhalb der H-Gase,

Fig. 9    ein Beispiel für die Wahl der Sensor-Outputfunktion im zweiten Ausführungsbeispiel zur Separierung wasserstoffreicher Gase (sog. "Hythanes"),

Fig. 10    ein Beispiel für die Wahl der Sensor-Outputfunktion im zweiten Ausführungsbeispiel zur Vorbereitung der Korrelation der Prandtl-Zahl innerhalb der L-Gase und

Fig. 11    ein Ausführungsbeispiel des schematischen Aufbaus einer Messvorrichtung gemäss vorliegender Erfindung.

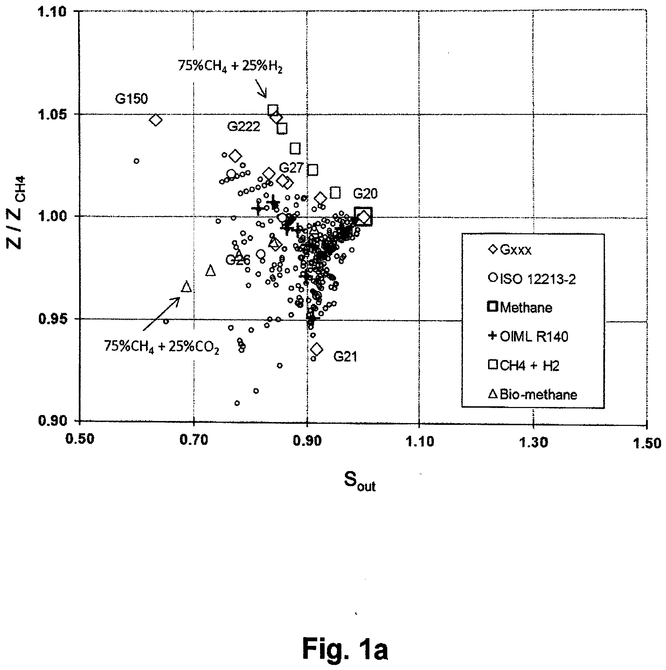

In allen in den Figuren 1a-10 gezeigten Darstellungen wird Methan (G20) wie folgt als Referenz verwendet:

$$\mu_i := \mu_{i,Gas} \, / \, \mu_{i,CH_4}$$

für alle Messgrössen $\mu_i$ in Gleichung (2) und allen darauffolgenden Abschnitten.

Ein erstes Ausführungsbeispiel des Verfahrens gemäss vorliegender Erfindung zur Korrelation des Realgasfaktors Z wird im Folgenden anhand der Figuren 1a - 5 beschrieben.

Das Verfahren geht von einer Auswahl oder Menge von Gasen und/oder Gasgemischen aus, für welche eine Gaseigenschaft Q bestimmt werden soll.

Zunächst wird versucht, die Sensor-Outputfunktion $S_{out}$ derart zu wählen, dass sich der Sensor-Output für alle Gase und/oder Gasgemische der Menge eindeutig auf die Gaseigenschaft Q, d.h. im ersten Ausführungsbeispiel auf den Realgasfaktor Z, abbilden lässt, beispielsweise, wie in Fig. 1a gezeigt, mittels der Sensor-Outputfunktion

$$S_{out} = f(c_p, c_s, \lambda) = c_p^{\ 1} \cdot c_s^{\ 1} \cdot \lambda^{-1}.$$

In einer grafischen Darstellung von Q in Abhängigkeit von $S_{out}$ bedeutet dies, dass die Punkte der Gase und/oder Gasgemische auf einer Linie oder zumindest näherungsweise auf einer Linie liegen, die sich durch eine eindeutige Funktion beschreiben lässt. Ist dies, wie in Fig. 1a gezeigt, nicht für alle Gase und/oder Gasgemische der Menge gleichzeitig möglich, kann durch Veränderung der Sensor-Outputfunktion $S_{out,1}$ visuell mitverfolgt werden, wie sich die Punkte der Gase und/oder Gasgemischen relativ zueinander verschieben. Fig. 1b zeigt die Verschiebung der Punkte aus Fig. 1a (eingezeichnete Verschiebungspfeile), wenn die Sensor-Outputfunktion beispielsweise in

$$S_{out,1} = f_1(c_p, c_s, \lambda) = c_p^{\ 1} \cdot c_s^{\ 1} \cdot \lambda^{0}$$

geändert wird (leere Symbole vor, gefüllte Symbole nach der Verschiebung).

Im Verfahren gemäss vorliegender Erfindung wird $S_{out}$ so verändert, dass eine Gruppe von Gasen und/oder Gasgemischen, im Folgenden Gasgemischgruppe genannt, entlang der Sensor-Outputachse vollständig von den übrigen Gasen und/oder Gasgemischen der Menge separiert wird, beispielsweise im ersten Ausführungsbeispiel durch die Sensor-Outputfunktion

$$S_{out,2} = f_2(c_p, c_s, \lambda) = c_p^{\ 1} \cdot c_s^{\ 1} \cdot \lambda^{0}.$$

Fig. 2a zeigt eine Darstellung des Realgasfaktors Z in Abhängigkeit von $S_{out,2}$ mit Methan (G20) als Referenz. Für die in Fig. 2 separierte Gasgemischgruppe der wasserstoffreichen Gase (sog. "Hythanes" der Form $CH_4 + H_2$) gilt Sensor-Output $S_{out,2} > 1$, und es kann, wie aus Fig. 2a ersichtlich, leicht eine Korrelationsfunktion für diese Gasgemischgruppe gefunden werden, weshalb diese im Folgenden nicht mehr betrachtet werden muss.

Ergibt die Messung dagegen $S_{out,2} \leq 1$, werden beispielsweise in einem nächsten Schritt durch erneutes Ändern der Sensor-Outputfunktion, z.B. in

$$S_{out,3} = f_3(c_p, c_s, \lambda) = c_p^{1} \cdot c_s^{-0.5} \cdot \lambda^{-1},$$

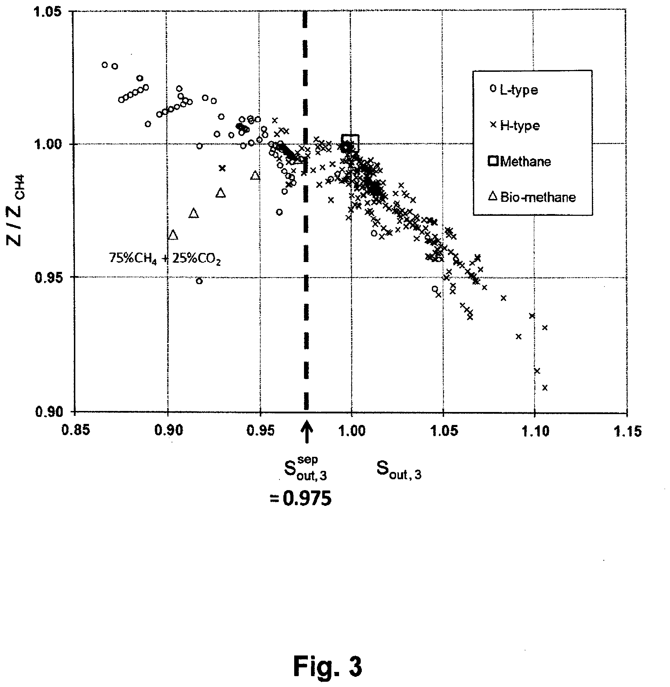

H- und L-Gase voneinander entlang der $S_{out,3}$-Achse getrennt. Fig. 3 zeigt die Trennung der H-Gase von den L-Gasen. Gilt Sensor-Output $S_{out,3} > 0.975$, handelt es sich um ein H-Gas. Für $S_{out,3} \leq 0.975$ handelt es sich um ein L-Gas. Im ersteren Fall wird zur Korrelation des Realgasfaktors Z für H-Gase übergegangen, im letzteren zur Korrelation des Realgasfaktors Z für L-Gase.

Zur Korrelation des Realgasfaktors Z für H-Gase kann beispielsweise die Sensor-Outputfunktion

$$S_{out,4} = f_4(c_p, c_s, \lambda) = c_p^{1} \cdot c_s^{-1} \cdot \lambda^{-1}$$

verwendet werden. Fig. 4 zeigt die Korrelation des Realgasfaktors Z in Abhängigkeit von $S_{out,4}$.

Zur Korrelation des Realgasfaktors Z für L-Gase kann beispielsweise die Sensor-Outputfunktion

$$S_{out,5} = f_5(c_p, c_s, \lambda) = c_p^{0.02} \cdot c_s^{-1.5} \cdot \lambda^{2}$$

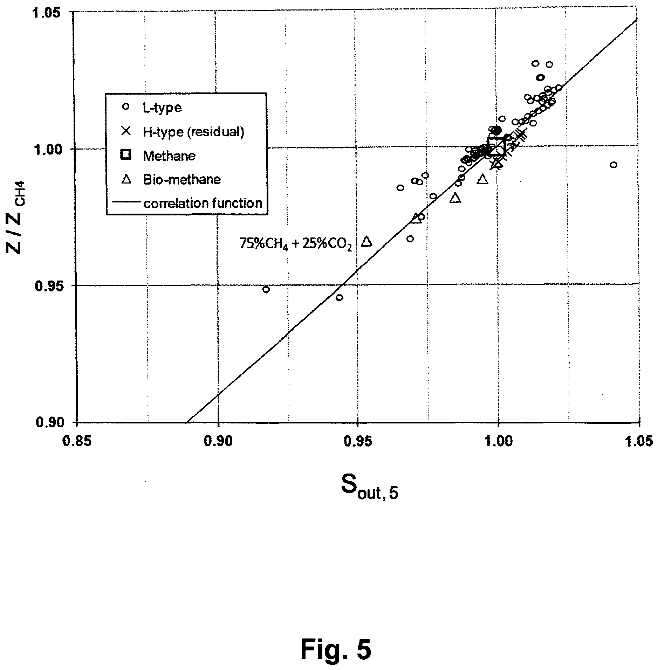

verwendet werden. Fig. 5 zeigt die Korrelation des Realgasfaktors Z in Abhängigkeit von $S_{out,5}$.

Alle in den Figuren 2, 4 und 5 gezeigten Korrelationsfunktionen $f_{corr,i}$ des ersten Ausführungsbeispiels sind für $i = 2, 4$ und 5 vom Typ

$$Z = f_{corr,i}(S_{out,i}) = a_{0,i} + a_{1,i} \cdot S_{out,i} + a_{2,i} \cdot S_{out,i}^{2} \quad (4),$$

d. h. Polymome zweiten Grades.

Welche Gasgemischgruppen voneinander getrennt werden können, wie die Sensor-Outputfunktionen $S_{out,i}$ lauten, wie viele Schritte benötigt werden, und wo genau die Trennlinien $S_{out,i}^{sep}$ gezogen werden können, hängt von den zur Verfügung stehenden Messgrössen $\mu_j$ und den zu korrelierenden Gaseigenschaften ab. Die oben genannten Werte sind lediglich beispielhaft.

Um zu zeigen, dass das Verfahren nicht auf den Realgasfaktor Z beschränkt ist, dass die zur Verfügung stehenden Messgrössen $\mu_j$ auch andere als die Wärmekapazität, die Schallgeschwindigkeit und die Wärmeleitfähigkeit sein können, und dass die verwendete Sensor-Outputfunktion $S_{out,i}$ bzw. die Korrelationsfunktion $f_{corr,i}$ nicht zwingend die Form des vorangehenden Beispiels haben müssen, sei hier in einem zweiten, verallgemeinernden Ausführungsbeispiel die Korrelation der Prandtl-Zahl Pr aufgeführt. Pr ist eine nach Ludwig Prandtl benannte dimensionslose Kennzahl von Fluiden, das heißt von Gasen oder Flüssigkeiten, und gibt das Verhältnis der Dicke der Strömungsgrenzschicht zur Dicke der Temperaturgrenzschicht in Wärmeübergangsproblemen an.

Bezüglich der Messgrössen $\mu_j$ im zweiten Ausführungsbeispiel wird auf die Veröffentlichung EP 2 806 271 A1 verwiesen. In dieser Veröffentlichung wird ein Verfahren zur Bestimmung physikalischer Gaseigenschaften beschrieben, in welchem das Gas oder Gasgemisch aus einem Gasreservoir unter Druck durch eine kritische Düse und über einen mikrothermischen Sensor fliesst, und der Druckabfall im Gasreservoir als Funktion der Zeit gemessen wird. Aus der Druckabfallmessung wird ein erster Gaseigenschaftsfaktor $\Gamma^*$ und aus dem Durchflusssignal des mikrothermischen Sensors ein zweiter Gaseigenschaftsfaktor $\Gamma$ bestimmt.

Der erste Gaseigenschaftsfaktor $\Gamma^*$ ist definiert als

$$\Gamma^* := C_d \cdot \psi_{max} \cdot \sqrt{\frac{1}{M}}, \quad (5)$$

worin $C_d$ den "Discharge Coefficient", d.h. den Verlustfaktor einer realen gegenüber einer idealen kritischen Düse, $M$ das Molekulargewicht des Gases und $\psi_{max}$ den Maximalwert der Ausflussfunktion bezeichnen.

Der zweite Gaseigenschaftsfaktor $\Gamma$ ist definiert als

$$\Gamma \;=\; \frac{c_p}{\lambda}\cdot C_d \cdot \psi_{max} \cdot \sqrt{M} \;,\qquad\qquad (6)$$

worin $c_p$ die Wärmekapazität und $\lambda$ die Wärmeleitfähigkeit bezeichnen.

Weiter wird mit Hilfe des mikrothermischen Sensors die Wärmeleitfähigkeit $\lambda$ des Gases oder Gasgemisches bestimmt und aus dem ersten und zweiten Gaseigenschaftsfaktor $\Gamma^*$, $\Gamma$ und der Wärmeleitfähigkeit $\lambda$ mittels Korrelation eine gesuchte physikalische Gaseigenschaft ermittelt.

Im zweiten Ausführungsbeispiel wird als Sensor-Outputfunktion

$$S_{out} = f(\Gamma^*,\Gamma,\lambda) = \alpha_1\cdot(\Gamma^*)^{\beta_1} + \alpha_2\cdot e^{(\Gamma-\Gamma_0)/\beta_2} + \alpha_3\cdot\tanh((\lambda-\lambda_0)/\beta_3)\qquad (7)$$

mit jeweils unterschiedlichen Konstanten $\alpha_1$, $\alpha_2$, $\alpha_3$, $\beta_1$, $\beta_2$, $\beta_3$, $\Gamma_0$ und $\lambda_0$ verwendet.

Die Korrelationsfunktion hat die Form

$$\mathrm{Pr} \;=\; f_{corr}(S_{out}) = a_1 + a_2\cdot(S_{out}-S_{out,0})^b\;,\qquad\qquad (8)$$

d.h. eine sogenannte Power-Funktion mit Offset $a_1$, Koeffizient $a_2$ und Exponenten $b$.

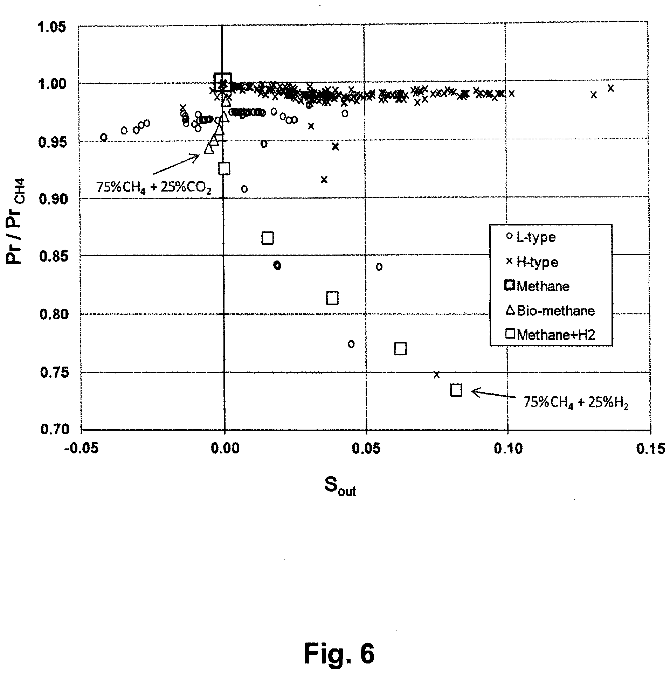

In einem 1. Schritt des zweiten Ausführungsbeispiels wird versucht, den Sensor-Output $S_{out}$ eindeutig auf die Prandtl-Zahl Pr abzubilden, beispielsweise, wie in Fig. 6 gezeigt, mittels der Sensor-Outputfunktion

$$S_{out} = \Gamma^* - e^{(\Gamma-1)} + \tanh(\lambda-1)\,.$$

Ist dies, wie in Fig. 6 gezeigt, nicht für alle Gase und/oder Gasgemische der Menge gleichzeitig möglich, können in einem 2. Schritt des Verfahrens die höherkalorischen H-Gase mittels der Sensor-Outputfunktion

$$S_{out,1} = f(\Gamma^*,\Gamma,\lambda) = (\Gamma^*)^5 + 0.125\cdot e^{(\Gamma-0.5)/0.5} + 0.125\cdot\tanh(\lambda-1)$$

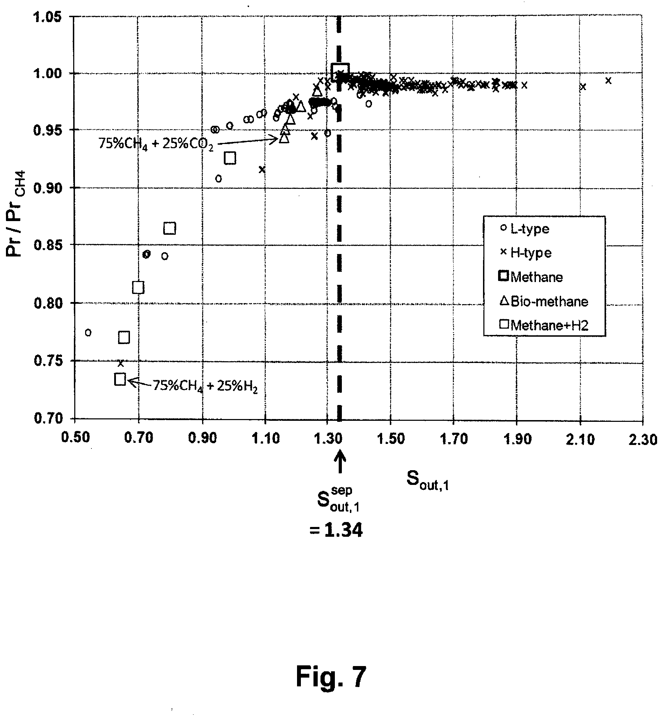

und des Grenzwertes $S_{out,1}^{sep} = 1.34$ separiert werden. Fig. 7 zeigt die Trennung der H-Gase von den übrigen Gasen und Gasgemischen.

In einem 3. Schritt des Verfahrens wird beispielsweise mittels

$$S_{out,2} = -0.46\cdot(\Gamma^*)^{3.74} - 0.865\cdot e^{(\Gamma-0.25)/0.83} - 2.26\cdot\tanh((\lambda-0.73)/1.43)$$

die Korrelation der Prandtl-Zahl Pr innerhalb der H-Gase vorbereitet und diese z.B. mittels der Korrelationsfunktion

$$\mathrm{Pr} \;=\; f_{corr}(S_{out,2}) = 0.98 + 0.012\cdot(S_{out,2}+3.82)^{-3.37}$$

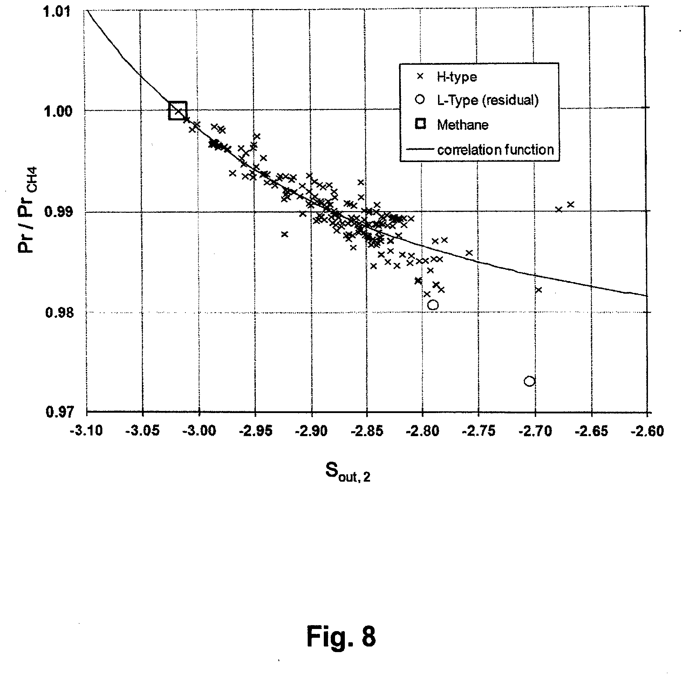

korreliert. Fig. 8 zeigt die Korrelation der Prandtl-Zahl Pr innerhalb der H-Gase in Abhängigkeit von $S_{out,2}$.

In einem 4. Schritt des Verfahrens werden beispielsweise mittels der Sensor-Outputfunktion

$$S_{out,3} = f(\Gamma^*,\Gamma,\lambda) = -4.44\cdot(\Gamma^*)^0 + 2\cdot e^{(\Gamma-0.5)/0.5} + \tanh(\lambda-1)$$

und des Grenzwertes $S_{out,3}^{sep} = 0.997$ die wasserstoffreichen Gase (sog. "Hythanes") separiert und die Prandtl-Zahl innerhalb der Hythanes mittels der Korrelationsfunktion

$$\mathrm{Pr} \ = \ f_{corr}(S_{out,3}) = 1.01 \cdot S_{out,3}^{-0.267}$$

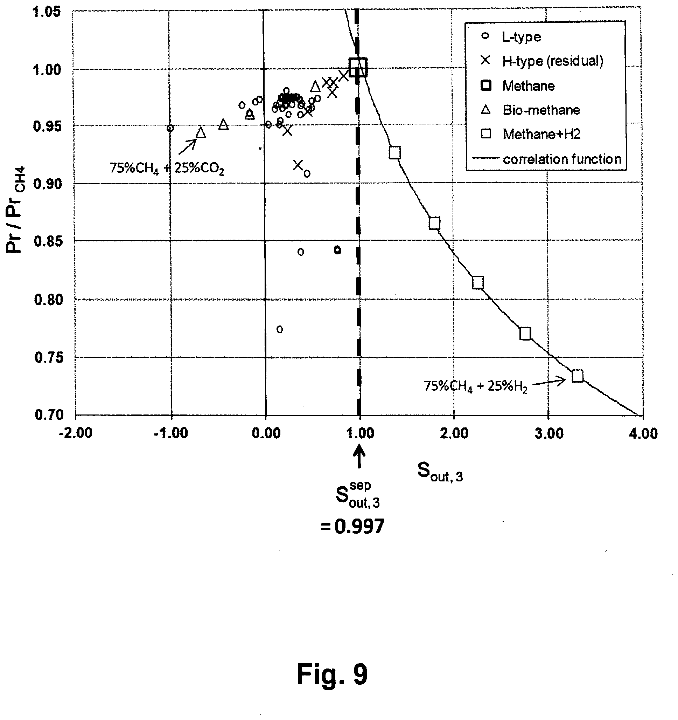

korreliert. Fig. 9 zeigt die Korrelation der Prandtl-Zahl Pr innerhalb der Hythanes in Abhängigkeit von $S_{out,3}$.

In einem 5. Schritt des Verfahrens werden beispielsweise mittels

$$S_{out,4} = f(\Gamma^{*}, \Gamma, \lambda) = (\Gamma^{*})^{2} + 0.5 \cdot e^{(\Gamma-1)/0.1} + 0.5 \cdot \tanh(\lambda + 1)$$

die Korrelation der Prandtl-Zahl innerhalb der L-Gase vorbereitet und diese z.B. mittels der Korrelationsfunktion

$$\mathrm{Pr} \ = \ f_{corr}(S_{out,4}) = 0.77 + 0.25 \cdot (S_{out,4} - 1.22)^{0.3}$$

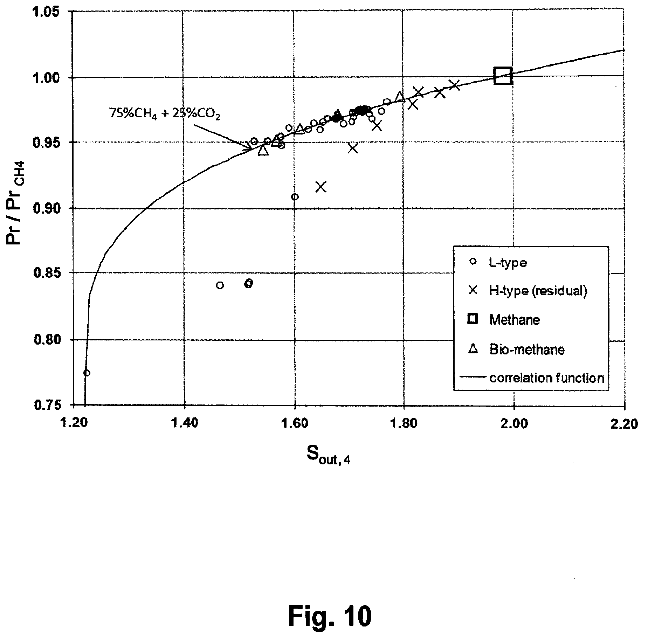

korreliert. Fig. 10 zeigt die Korrelation der Prandtl-Zahl Pr innerhalb der L-Gase in Abhängigkeit von $S_{out,4}$.

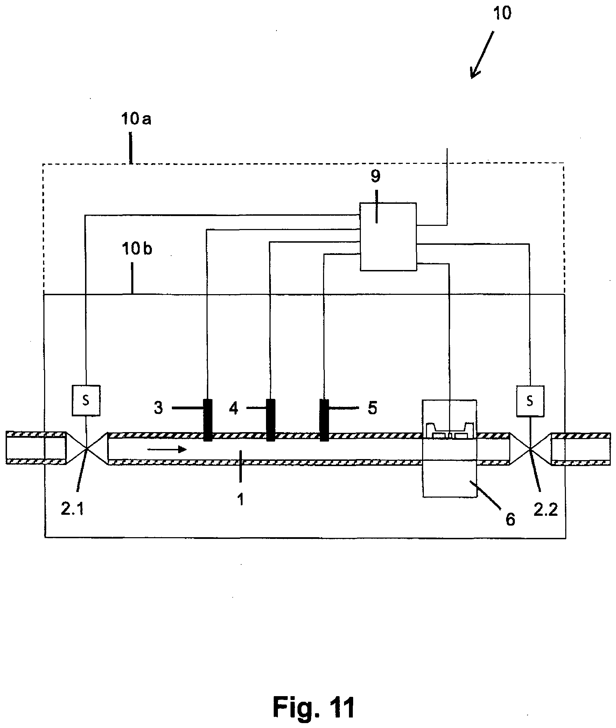

Fig. 11 zeigt ein Ausführungsbeispiel des schematischen Aufbaus einer Messvorrichtung gemäss vorliegender Erfindung. Im Ausführungsbeispiel umfasst die Messvorrichtung 10 einen oder mehrere Sensoren 3, 4, 5, 6 zur Erfassung von physikalischen Messgrössen $\mu_j$ ($j = 1,...,m$) und eine Auswerteeinheit 9, die zur Ausführung eines Verfahrens gemäss vorliegender Erfindung oder einer der oben beschriebenen Ausführungsformen oder -varianten des Verfahrens eingerichtet ist. Als Sensoren können beispielsweise einer oder mehrere der folgenden Sensoren vorgesehen sein: ein mikrothermischer Sensor 6, ein Ultraschalldurchflusssensor 5, ein Temperatursensor 4, ein Drucksensor 3 oder ein anderer passender Sensor. In einer vorteilhaften Ausführungsvariante sind die Sensoren in einer Gasleitung 1 angeordnet.

Einzelne dieser Komponenten oder alle diese Komponenten können zu einer Baueinheit zusammengefasst werden, wobei die Auswerteeinheit 9 Bestandteil dieser Baueinheit sein kann (Variante 10a), oder die Auswerteeinheit kann separat dazu beigestellt werden (Variante 10b), z.B. in einer übergeordneten Recheneinheit.

Bei Bedarf kann die Messvorrichtung 10 zusätzliche Komponenten enthalten, wie zum Beispiel ein oder mehrere Absperrventile 2.1, 2.2. Mit Hilfe der Absperrventile ist es möglich, eine oder mehrere der physikalischen Messgrössen wahlweise unter Durchfluss oder ruhend zu erfassen.

Das oben beschriebene Verfahren und die oben beschriebenen Ausführungsformen und -varianten sowie die oben beschriebene Messvorrichtung eignen sich beispielsweise für die Bestimmung von Gaseigenschaften von brennbaren Gasen und/oder Gasgemischen und/oder von Gasen und/oder Gasgemischen aus dem Energiebereich.

Das Verfahren und die Messvorrichtung gemäss vorliegender Erfindung haben den Vorteil, dass dank der Korrelation in mehreren Schritten die Genauigkeit der Bestimmung von Gaseigenschaften aus physikalischen Messwerten der Gase und/oder Gasgemische verbessert werden kann, und dass die Menge der Gase und/oder Gasgemische, für die das Verfahren mit der gewünschten Genauigkeit angewendet werden kann, grösser ist als bei einfachen Korrelationsverfahren. Ein weiterer Vorteil besteht darin, dass die vorgestellte Messvorrichtung vergleichsweise günstig hergestellt werden kann, was eine wirtschaftliche Bestimmung von Gaseigenschaften vor Ort ermöglicht.

**Patentansprüche**

1. Verfahren zur Bestimmung von Gaseigenschaften mittels Korrelation, in welchem

- eine zu bestimmende Gaseigenschaft (Q) aus physikalischen Messgrössen ($\mu_j$ ($j = 1,...,m$)) der Gase und/oder Gasgemische mittles Korrelation bestimmt wird, **dadurch gekennzeichnet, dass**
- die physikalischen Messgrössen ($\mu_j$ ($j = 1,...,m$)) mittels einer Sensor-Outputfunktion ($f$) zu einem Sensor-Output ($S_{out} = f(\mu_1,..., \mu_m)$) zusammengefasst werden,
- der Sensor-Output ($S_{out}$) mit einem Grenzwert $(S_{out}^{sep})$ verglichen wird, um festzustellen, ob der Sensor-Output innerhalb der Menge (G) von Gasen und/oder Gasgemischen, auf die das Verfahren Anwendung findet, zu einer Gruppe von Gasen und/oder Gasgemischen, im Folgenden Gasgemischgruppe (GG) genannt, gehört, in welcher die Korrelation zwischen dem Sensor-Output ($S_{out}$) und der zu bestimmenden Gaseigenschaft (Q) besser ist als in der gesamten Menge (G), und

- wenn der Sensor-Output ($S_{out}$) zur genannten Gasgemischgruppe (GG) gehört, die Gaseigenschaft (Q) mittels einer Korrelationsfunktion ($f_{corr}$), die für die Gasgemischgruppe spezifisch ist, aus dem Sensor-Output bestimmt wird.

2. Verfahren nach Anspruch 1, wobei die Zugehörigkeit zu einer Gasgemischgruppe in zwei, drei, vier oder mehr Schritten geprüft wird,

- indem jeweils die physikalischen Messgrössen ($\mu_j$ (j = 1,...,m)) mittels einer Sensor-Outputfunktion ($f_i$), die für die nach Separierung der vorangehenden Gasgemischgruppe oder -gruppen übrig gebliebenen Gase und/oder Gasgemische ($G_{rest,i}$) der Menge, im Folgenden übrig gebliebene Gase und/oder Gasgemische ($G_{rest,i}$) genannt, spezifisch ist, zu einem weiteren Sensor-Output ($S_{out,i} = f_i(\mu_1,..., \mu_m)$) zusammengefasst werden, und der weitere Sensor-Output ($S_{out,i}$) mit einem weiteren Grenzwert $\left( S_{out,i}^{sep} \right)$ verglichen wird, um festzustellen, ob der weitere Sensor-Output innerhalb der übrig gebliebenen Gase und/oder Gasgemische ($G_{rest,i}$) zu einer weiteren Gasgemischgruppe ($GG_i$) gehört, in welcher die Korrelation zwischen dem weiteren Sensor-Output ($S_{out,i}$) und der zu bestimmenden Gaseigenschaft (Q) besser ist als innerhalb der übrig gebliebenen Gase und/oder Gasgemische ($G_{rest,i}$), und

- wenn der weitere Sensor-Output ($S_{out,i}$) zur genannten weiteren Gasgemischgruppe ($GG_i$) gehört, die Gaseigenschaft (Q) mittels einer für die weitere Gasgemischgruppe spezifischen Korrelationsfunktion ($f_{corr,i}$) aus dem weiteren Sensor-Output bestimmt wird.

3. Verfahren nach Anspruch 1 oder 2, in welchem, wenn der Sensor-Output ($S_{out}$, $S_{out,i}$) nicht zu einer der genannten Gasgemischgruppen (GG, $GG_i$) gehört, die Gaseigenschaft (Q) mittels einer Korrelationsfunktion ($f_{corr,i}$), die für die übriggebliebenen Gase und/oder Gasgemische ($G_{rest}$, $G_{rest,i}$) spezifisch ist, aus dem Sensor-Output bestimmt wird.

4. Verfahren nach einem der Ansprüche 1 bis 3, wobei der Sensor-Output ($S_{out}$, $S_{out,i}$) vor der Korrelation mit einer weiteren Sensor-Outputfunktion ($f_i$) verändert wird.

5. Verfahren nach einem der Ansprüche 1 bis 4, in welchem die Sensor-Outputfunktion ($f$) und ein Grenzwert $\left( S_{out}^{sep} \right)$ für den Sensor-Output derart festgelegt werden, dass mittels desselben aus einer Menge (G) von Gasen und/oder Gasgemischen, für welche die Gaseigenschaft (Q) bestimmt wird, eine Gasgemischgruppe (GG) separiert wird, innerhalb welcher die Korrelation zwischen dem Sensor-Output ($S_{out}$) und der Gaseigenschaft (Q) besser ist als in der gesamten Menge (G).

6. Verfahren nach einem der Ansprüche 1 bis 5, in welchem für die Gase und/oder Gasgemische der Gasgemischgruppe (GG) die Beziehung

$$S_{out}^{GG} < S_{out}^{sep}$$

und für die übrigen Gase und/oder Gasgemische ($G_{rest}$) der Menge die Beziehung

$$S_{out}^{G\,rest} \geq S_{out}^{sep},$$

bzw. fallweise, an Stelle der genannten Beziehungen, die Beziehungen $S_{out}^{GG} > S_{out}^{sep}$ und $S_{out}^{G\,rest} \leq S_{out}^{sep}$ gelten.

7. Verfahren nach einem der Ansprüche 1 bis 6, welches automatisch ausgeführt wird, zum Beispiel automatisch in einer Messvorrichtung.

8. Verfahren nach einem der Ansprüche 1 bis 7, in welchem die Sensor-Outputfunktion oder Sensor-Outputfunktionen ($f, f_i$) und/oder der oder die Grenzwerte ( $S_{out}^{sep}$, $S_{out,i}^{sep}$ ) für den Sensor-Output und/oder die Korrelationsfunktionen

($f_{corr}$, $f_{corr,i}$) vorab festgelegt werden, zum Beispiel anhand von Werten der physikalischen Messgrössen und der zu bestimmenden Gaseigenschaft aus Tabellenwerken und/oder Fachliteratur und/oder Datenbanken und/oder Messungen.

9.  Verfahren nach einem der Ansprüche 1 bis 8, in welchem die Sensor-Outputfunktion oder Sensor-Outputfunktionen ($f$, $f_i$) und/oder der oder die Grenzwerte ($S_{out}^{sep}$, $S_{out,i}^{sep}$) für den Sensor-Output mit einem Computerprogramm ermittelt werden, indem

- für jede Funktion ($f_i$) in einer Menge möglicher Sensor-Output-Funktionen die jeweiligen Funktionsparameter ($p_{fi}$) von ($f_i$), wie beispielsweise Polynomkoeffizienten, Exponenten oder Konstanten, innerhalb vorgegebener Grenzwerte für ($p_{fi}$) variiert werden, z.B. mittels eines Monte-Carlo-Auswahlverfahrens,
- der Sensor-Output-Bereich in Intervalle unterteilt und insbesondere in jedem Intervall die Anzahl Doppeldeutigkeiten gezählt wird, d.h. die Anzahl Ereignisse, für die zwei oder mehrere Gasgemische unterschiedliche Werte für die zu korrelierende Grösse (Q) zeigen oder die Werte für die zu korrelierende Grösse (Q) ausserhalb eines vorgegebenen Werteintervalls für (Q) liegen,
- diejenige Funktion ($f_i$) und derjenige Funktionsparametersatz ($p_{fi}$) bestimmt wird, bei welchen am wenigsten solche Doppeldeutigkeitsereignisse auftreten, oder die Streuung der Werte für die zu korrelierende Grösse (Q) bei Doppeldeutigkeitsereignissen in einem Intervall minimal ist, oder eine vorgegebene maximal erlaubte Anzahl ($n_{max}$) der Doppeldeutigkeitsereignisse bzw. eine vorgegebene maximal erlaubte Streuung der Q-Werte bei Doppeldeutigkeitsereignissen in einem Intervall nicht überschritten wird, und

indem insbesondere ermittelt wird, ab welchem Grenzwert ($S_{out,i}^{sep}$) eine je Intervall für die Ermittlung des Grenzwertes vorgegebene Anzahl ($\underline{n}_{max}$) der Doppeldeutigkeitsereignisse bzw. eine je Intervall für die Ermittlung des Grenzwertes vorgegebene Streuung der Q-Werte bei Doppeldeutigkeitsereignissen nicht überschritten wird.

10. Verfahren nach einem der Ansprüche 1 bis 9, wobei mindestens zwei oder alle Korrelationsfunktionen verschieden voneinander sind, und/oder wobei die Punkte der Gase und/oder Gasgemische der Gasgemischgruppe oder -gruppen jeweils auf einer durch eine eindeutige Korrelationsfunktion beschriebenen Linie oder in Toleranzbereichen liegen, die beidseits an eine derartige Linie anschliessen, und die zum Beispiel nicht grösser als 0.25 % oder 0.75 % oder 2 % des Wertes der Gaseigenschaft (Q) sind.

11. Verfahren nach einem der Ansprüche 1 bis 10, wobei die Sensor-Outputfunktion ($f$) oder Sensor-Outputfunktionen ($f_i$) vom Typ

$$S_{out,i} = \mu_1^{p_{1,i}} \cdot ... \cdot \mu_m^{p_{m,i}}$$

und $p_{1,i}$, ..., $p_{m,i}$ Exponenten sind, und/oder
wobei die Korrelationsfunktion ($f_{corr}(S_{out})$) oder Korrelationsfunktionen ($f_{corr,i}(S_{out,i})$) vom Typ

$$Q = f_{corr,i}(S_{out,i}) = a_{0,i} + a_{1,i} \cdot S_{out,i} + a_{2,i} \cdot S_{out,i}^2$$

und $a_{0,i}$, $a_{1,i}$ und $a_{2,i}$ Konstanten sind.

12. Verfahren nach einem der Ansprüche 1 bis 11, wobei als Mass für die Genauigkeit der Korrelation der Pearson Korrelationskoeffizient ($Kor(S_{out}, Q)$) verwendet wird, und wobei eine bessere Korrelation bedeutet, dass der Pearson Korrelationskoeffizient näher beim Wert 1 oder -1 liegt, insbesondere dass der Absolutwert der Differenz zum Wert 1 bzw. -1 kleiner als 0.3 oder 0.2 oder 0.1 ist.

13. Verfahren nach einem der Ansprüche 1 bis 12, wobei die physikalischen Messgrössen ($\mu_j$ ($j = 1,...,m$)) mit einem oder mehreren Sensoren erfasst werden, und/oder wobei als physikalische Messgrössen ($\mu_j$ ($j = 1,... ,m$)) mindestens zwei der Messgrössen Wärmeleitfähigkeit, Wärmekapazität, Wärmediffusivität, Dichte, Strömungsgeschwindigkeit, Massenfluss, Schallgeschwindigkeit, Dielektrizitätskonstante, Viskosität, Infrarot-Absorption, Druck oder Temperatur erfasst werden.

14. Messvorrichtung zur Bestimmung von Gaseigenschaften mit einem oder mehreren Sensoren (3, 4, 5, 6) zur Erfassung von physikalischen Messgrössen ($\mu_j$ (j = 1,...,m)) und mit einer Auswerteeinheit (9), **dadurch gekennzeichnet, dass** die Auswerteeinheit zur Ausführung eines Verfahrens gemäss einem der Ansprüche 1 bis 13 eingerichtet ist.

15. Messvorrichtung nach Anspruch 14, in welcher die Auswerteeinheit zusammen mit dem oder den Sensoren eine Baueinheit bilden, oder in welcher die Auswerteeinheit in einer separaten Recheneinheit ausgebildet ist.

**Claims**

1. A method for the determination of gas properties by correlation in which

   - a gas property (Q) is determined by correlation from physical measuring quantities ($\mu_j$ (j = 1,...,m)) of the gases and/or gas mixtures,
   **characterized in that**
   - the physical measuring quantities ($\mu_j$ (j = 1,...,m)) are combined into a sensor output ($S_{out} = f(\mu_1,...,\mu_m)$) by making use of a sensor output function (f),

   - the sensor output ($S_{out}$) is compared with a limit value $\left( S_{out}^{sep} \right)$ in order to determine whether the sensor output within the set (G) of gases and/or gas mixtures to which the method is applied belongs to a group of gases and/or gas mixtures, which are referred to below as gas mixture group (GG), in which the correlation between the sensor output ($S_{out}$) and the gas property (Q) to be determined is better than in the entire set (G), and
   - if the sensor output ($S_{out}$) belongs to the said gas mixture group (GG), the gas property (Q) is determined from the sensor output with a correlation function ($f_{corr}$) which is specific to the gas mixture group.

2. A method according to claim 1, wherein the affiliation with a gas mixture group is checked in two, three, four or more steps,

   - in that the physical measuring quantities ($\mu_j$ (j = 1,...,m)) are combined into a further sensor output ($S_{out,i} = f_i(\mu_1,..., \mu_m)$) respectively by making use of a sensor output function ($f_i$) which is specific to the gases and/or gas mixtures ($G_{rest,i}$) of the set that have remained after the separation of the preceding gas mixture group or groups, which are referred to below as the remaining gases and/or gas mixtures ($G_{rest,i}$), and the further sensor output ($S_{out,i}$) is compared with a further limit value $\left( S_{out,i}^{sep} \right)$ in order to determine whether the further sensor output belongs within the remaining gases and/or gas mixtures ($G_{rest,i}$) to a further gas mixture group ($GG_i$) in which the correlation between the further sensor output ($S_{out,i}$) and the gas property (Q) to be determined is better than within the remaining gases and/or gas mixtures ($G_{rest,i}$), and
   - if the further sensor output ($S_{out,i}$) belongs to the said further gas mixture group ($GG_i$), the gas property (Q) is determined from the further sensor output with a correlation function ($f_{corr,i}$) which is specific to the further gas mixture group.

3. A method according to claim 1 or 2, wherein, if the sensor output ($S_{out}$, $S_{out,i}$) does not belong to one of the said gas mixture groups (GG, $GG_i$), the gas property (Q) is determined from the sensor output with a correlation function ($\underline{f_{corr,i}}$) which is specific to the remaining gases and/or gas mixtures ($G_{rest}$, $G_{rest,i}$).

4. A method according to one of the claims 1 to 3, wherein the sensor output ($S_{out}$, $S_{out,i}$) is changed before the correlation with a further sensor output function ($\underline{f}_i$).

5. A method according to one of the claims 1 to 4, wherein the sensor output function (f) and a limit value $\left( S_{out}^{sep} \right)$ for the sensor output are determined in such a way that a gas mixture group (GG) is separated by the limit value from a set (G) of gases and/or gas mixtures for which the gas property (Q) is determined, within which the correlation between the sensor output ($s_{out}$) and the gas property (Q) is better than in the entire set (G).

6. A method according to one of the claims 1 to 5, wherein the relationship

**13**

$$S_{out}^{GG} < S_{out}^{sep}$$

applies to the gases and/or gas mixtures of the gas mixture group (GG), and the relationship

$$S_{out}^{Grest} \geq S_{out}^{sep},$$

applies to the remaining gases and/or gas mixtures ($G_{rest}$) of the set, or from case to case, instead of the aforementioned relationships, the relationships $S_{out}^{GG} > S_{out}^{sep}$ and $S_{out}^{Grest} \leq S_{out}^{sep}$ apply.

**7.** A method according to one of the claims 1 to 6, which is carried out automatically, e.g. automatically in a measuring apparatus.

**8.** A method according to one of the claims 1 to 7, wherein the sensor output function or sensor output functions ($f$, $f_i$) and/or the limit value or values ( $S_{out}^{sep}$ , $S_{out,i}^{sep}$ ) for the sensor output and/or the correlation functions ($f_{corr}$, $f_{corr,i}$) are determined in advance, e.g. on the basis of values of the physical measuring quantities and the gas property to be determined from tables and/or technical literature and/or databases and/or measurements.

**9.** A method according to one of the claims 1 to 8, wherein the sensor output function or the sensor output functions ($f$, $f_i$) and/or the limit value or values ( $S_{out}^{sep}$ , $S_{out,i}^{sep}$ ) for the sensor output are determined by a computer program in that

- for each function ($f_i$) in a set of possible sensor output functions the respective functional parameters ($p_{fi}$) of ($f_i$) such as polynomial coefficients, exponents or constants, are varied within preset limit values for ($p_{fi}$), e.g. by means of a Monte Carlo selection method,
- the sensor output range is subdivided into intervals and the number of ambiguities is counted in particular in each interval, i.e. the number of events for which two or more gas mixtures show different values for the quantity (Q) to be determined or the values for the quantity (Q) to be determined lie outside of a preset value interval for (Q),
- the function ($f_i$) and the specific functional parameter set ($p_{fi}$) are determined for which the fewest of such ambiguity events occur, or the variance of the values for the quantity (Q) to be determined is minimal in case of ambiguity events in an interval, or a preset maximum permitted number ($n_{max}$) of the ambiguity events or a preset maximum permitted variance of the Q values is not exceeded in case of ambiguity events in an interval, and

in that it is determined in particular from which limit value $\left( S_{out,i}^{sep} \right)$ a number ($\underline{n}_{max}$) of the ambiguity events preset for each interval for the determination of the limit value or a variance of the Q values preset for each interval for the determination of the limit value is not exceeded in case of ambiguity events.

**10.** A method according to one of the claims 1 to 9, wherein at least two or all correlation functions differ from each other, and/or wherein the points of the gases and/or gas mixtures of the gas mixture group or groups each lie on a line described by a distinct correlation function or in tolerance ranges which adjoin such a line on both sides, and which for example are not greater than 0.25% or 0.75% or 2% of the value of the gas property (Q).

**11.** A method according to one of the claims 1 to 10, wherein the sensor output function ($f$) or sensor output functions ($f_i$) are of the type

$$S_{out,i} = \mu_1^{p_{1,i}} \cdot ... \cdot \mu_m^{p_{m,i}}$$

and $p_{1,i}$ ..., $p_{m,i}$ are exponents, and/or
wherein the correlation function ($f_{corr}(S_{out})$) or correlation functions ($f_{corr,i}(S_{out,i})$) are of the type

$$Q = f_{corr,i}(S_{out,i}) = a_{0,i} + a_{1,i} \cdot S_{out,i} + a_{2,i} \cdot S_{out,i}^{2}$$

and $a_{0,i}$, $a_{1,i}$ and $a_{2,i}$ are constants.

12. A method according to one of the claims 1 to 11, wherein the Pearson correlation coefficient ($Kor(S_{out},Q)$) is used as a measure for the precision of the correlation, and wherein a better correlation means that the Pearson correlation coefficient lies closer to the value 1 or -1, in particular that the absolute value of the difference from the value 1 or -1 is less than 0.3 or 0.2 or 0.1.

13. A method according to one of the claims 1 to 12, wherein the physical measuring quantities ($\mu_j$ (j = 1,...,m)) are detected with one or several sensors, and/or wherein at least two of the measuring quantities thermal conductivity, heat capacity, thermal diffusivity, density, flow velocity, mass flow, sound velocity, dielectric constant, viscosity, infrared absorption, pressure or temperature are detected as physical measuring quantities ($\mu_j$ (j = 1,...,m)).

14. A measuring apparatus for determining gas properties with one or several sensors (3, 4, 5, 6) for detecting physical measuring quantities ($\mu_j$ (j = 1,...,m)) and with an evaluation unit (9), **characterized in that** the evaluation unit is set up for carrying out a method according to one of the claims 1 to 13.

15. A measuring apparatus according to claim 14, wherein the evaluation unit forms an assembly together with the sensor or sensors, or wherein the evaluation unit is formed in a separate computing unit.

**Revendications**

1. Procédé pour déterminer des propriétés de gaz au moyen d'une corrélation, dans lequel

- une propriété de gaz à déterminer (Q) est déterminée au moyen d'une corrélation à partir de grandeurs de mesure physiques ($\mu_j$ (j = 1,...,m)) des gaz et/ou des mélanges de gaz,
**caractérisé en ce que**
- les grandeurs de mesure physiques ($\mu_j$ (j = 1,...,m)) sont réunies au moyen d'une fonction de sortie de capteur (f) en une sortie de capteur ($S_{out} = f(\mu_1,...,\mu_m)$),

- la sortie de capteur ($S_{out}$) est comparée à une valeur limite $\left( S_{out}^{sep} \right)$ afin de voir si la sortie de capteur appartient, dans la quantité (G) de gaz et/ou de mélanges de gaz à laquelle le procédé est appliqué, à un groupe de gaz et ou de mélanges de gaz, appelé ci-après groupe de mélanges de gaz (GG), dans lequel la corrélation entre la sortie de capteur ($S_{out}$) et la propriété de gaz à déterminer (Q) est meilleure que dans la quantité (G) entière et,
- si la sortie de capteur ($S_{out}$) appartient audit groupe de mélanges de gaz (GG), la propriété de gaz (Q) est déterminée au moyen d'une fonction de corrélation ($f_{corr}$) propre au groupe de mélanges de gaz à partir de la sortie de capteur.

2. Procédé selon la revendication 1, dans lequel l'appartenance à un groupe de mélanges de gaz est vérifiée en deux, trois, quatre étapes

- en réunissant les grandeurs de mesure physiques ($\mu_j$ (j = 1,...,m)) chaque fois au moyen d'une fonction de sortie de capteur ($f_i$) propre aux gaz et/ou mélanges de gaz de la quantité qui restent après la séparation du ou des groupes de mélanges de gaz antérieurs, appelés ci-après gaz et/ou mélanges de gaz restants ($G_{rest,i}$), dans une autre sortie de capteur ($S_{out,i} = f_i(\mu_1,...,\mu_m)$), et en comparant l'autre sortie de capteur ($S_{out,i}$) à une autre valeur limite $\left( S_{out,i}^{sep} \right)$ pour voir si l'autre sortie de capteur appartient, parmi les gaz et/ou mélanges de gaz restants ($G_{rest,i}$), à un autre groupe de mélanges de gaz ($GG_i$) dans lequel la corrélation entre l'autre sortie de capteur ($S_{out,i}$) et la propriété de gaz à déterminer (Q) est meilleure que parmi les gaz et/ou mélanges de gaz restants ($G_{rest,i}$), et
- si l'autre sortie de capteur ($S_{out,i}$) appartient audit autre groupe de mélanges de gaz ($GG_i$), la propriété de gaz (Q) est déterminée au moyen d'une fonction de corrélation ($f_{corr,i}$) propre à l'autre groupe de mélanges de gaz à partir de l'autre sortie de capteur.

**3.** Procédé selon la revendication 1 ou 2 dans lequel, si la sortie de capteur ($S_{out}$, $S_{out,i}$) n'appartient pas à l'un desdits groupes de mélanges de gaz (GG, $GG_i$), la propriété de gaz (Q) est déterminée a partir de la sortie de capteur au moyen d'une fonction de corrélation ($\underline{f_{corr,i}}$) propre aux gaz et/ou mélanges de gaz restants ($G_{rest}$, $G_{rest,i}$).

**4.** Procédé selon l'une des revendications 1 à 3, dans lequel la sortie de capteur ($S_{out}$, $S_{out,i}$) est modifiée avant la corrélation avec une autre fonction de sortie de capteur ($\underline{f_i}$).

**5.** Procédé selon l'une des revendications 1 à 4, dans lequel la fonction de sortie de capteur ($f$) et une valeur limite $\left( S_{out}^{sep} \right)$ pour la sortie de capteur sont fixées de telle façon qu'elles permettent de séparer, à partir d'une quantité (G) de gaz et/ou de mélanges de gaz pour laquelle la propriété de gaz (Q) est déterminée, un groupe de mélanges de gaz (GG) dans lequel la corrélation entre la sortie de capteur ($S_{out}$) et la propriété de gaz (Q) est meilleure que dans la quantité (G) entière.

**6.** Procédé selon l'une des revendications 1 à 5 dans lequel, pour les gaz et/ou mélanges de gaz du groupe de mélanges de gaz (GG), la relation

$$S_{out}^{GG} < S_{out}^{sep}$$

s'applique, et pour les autres gaz et/ou mélanges de gaz ($G_{rest}$) de la quantité, la relation

$$S_{out}^{Grest} \geq S_{out}^{sep},$$

s'applique ou, dans certains cas, les relations $S_{out}^{GG} > S_{out}^{sep}$ et $S_{out}^{Grest} \leq S_{out}^{sep}$ s'appliquent au lieu de lesdites relations précédentes.

**7.** Procédé selon l'une des revendications 1 à 6 qui est exécuté automatiquement, par exemple automatiquement dans un dispositif de mesure.

**8.** Procédé selon l'une des revendications 1 à 7, dans lequel la fonction de sortie de capteur ou les fonctions de sortie de capteur ($f$, $f_i$) et/ou la ou les valeurs limites ( $S_{out}^{sep}$, $S_{out,i}^{sep}$ ) de la sortie de capteur et/ou les fonctions de corrélation ($f_{corr}$, $f_{corr,i}$) sont définies à l'avance, par exemple à l'aide de valeurs des grandeurs de mesure physiques et de la propriété de gaz à déterminer issues de tableaux de référence et/ou de la littérature spécialisée et/ou de bases de données et/ou de mesures.

**9.** Procédé selon l'une des revendications 1 à 8, dans lequel la fonction de sortie de capteur ou les fonctions de sortie de capteur ($f$, $f_i$) et/ou la ou les valeurs limites ( $S_{out}^{sep}$, $S_{out,i}^{sep}$ ) de la sortie de capteur sont déterminées avec un programme d'ordinateur, dans lequel

- pour chaque fonction ($f_i$) dans une quantité de fonctions de sortie de capteur possibles, les paramètres de fonction ($p_{fi}$) correspondants de ($f_i$), par exemple des coefficients polynomiaux, des exposants ou des constantes, sont variés dans des limites prédéterminées pour ($p_{fi}$), par exemple au moyen d'une méthode de sélection de Monte Carlo,
- la plage de sortie de capteur est divisée en intervalles et, en particulier, le nombre d'ambiguïtés dans chaque intervalle est compté, c'est-à-dire le nombre d'événements qui présentent des valeurs différentes de la grandeur à corréler (Q) pour deux ou plusieurs mélanges de gaz, ou les valeurs de la grandeur à corréler (Q) qui se situent en dehors d'un intervalle de valeurs prédéterminé pour (Q),
- la fonction ($f_i$) et l'ensemble de paramètres de fonction ($p_{fi}$) pour lesquels ces résultats d'ambiguïté sont les moins nombreux ou la dispersion des valeurs de la grandeur à corréler (Q) en cas d'événements d'ambiguïté dans un intervalle est minimale, ou pour lesquels un nombre maximal autorisé ($n_{max}$) prédéfini d'événements d'ambiguïté ou une dispersion maximale autorisée prédéfinie des valeurs de Q en cas d'événements d'ambiguïté dans un intervalle ne sont pas dépassés, sont déterminés et

le programme détermine, en particulier, à partir de quelle valeur limite $\left( S_{out,i}^{sep} \right)$ un nombre ($n_{max}$) d'événements d'ambiguïté prédéterminé pour la détermination de la valeur limite pour chaque intervalle ou une dispersion des valeurs de Q en cas d'événements d'ambiguïté prédéterminée pour chaque intervalle pour la détermination de la valeur limite n'est pas dépassé.

**10.** Procédé selon l'une des revendications 1 à 9, dans lequel au moins deux ou toutes les fonctions de corrélation sont différentes les unes des autres et/ou dans lequel les points des gaz et/ou mélanges de gaz du ou des groupes de mélanges de gaz se situent sur une ligne décrite par une fonction de corrélation univoque ou dans des plages de tolérances situées de part et d'autre d'une telle ligne et qui ne dépassent pas, par exemple, 0,25 % ou 0,75 % ou 2 % de la valeur de la propriété de gaz (Q).

**11.** Procédé selon l'une des revendications 1 à 10, dans lequel la fonction de sortie de capteur (*f*) ou les fonctions de sortie de capteur (*f$_i$*) sont du type

$$ S_{out,i} = \mu_1^{p_{1,i}} \cdot \ldots \cdot \mu_m^{p_{m,i}} $$

et $p_{1,i}$ ..., $p_{m,i}$ sont des exposants, et/ou
dans lequel la fonction de corrélation ($f_{corr}(S_{out})$) ou les fonctions de corrélation ($f_{corr,i}(S_{out,i})$) sont du type

$$ Q = f_{corr,i}(S_{out,i}) = a_{0,i} + a_{1,i} \cdot S_{out,i} + a_{2,i} \cdot S_{out,i}^2 $$

et $a_{0,i}$, $a_{1,i}$ et $a_{2,i}$ sont des constantes.

**12.** Procédé selon l'une des revendications 1 à 11, dans lequel la mesure de la précision de la corrélation est donnée par le coefficient de corrélation de Pearson ($Kor(S_{out},Q)$) et dans lequel une meilleure corrélation signifie que le coefficient de corrélation de Pearson est plus proche de la valeur 1 ou -1, en particulier que la valeur absolue de la différence par rapport à la valeur 1 ou -1 est inférieure à 0,3 ou 0,2 ou 0,1.

**13.** Procédé selon l'une des revendications 1 à 12, dans lequel les grandeurs de mesure physiques ($\mu_j$ *(j = 1,...,m)*) sont acquises avec un ou plusieurs capteurs et/ou dans lequel les grandeurs physiques mesurées ($\mu_j$ *(j = 1,...,m)*) sont au moins deux des grandeurs de conductibilité thermique, capacité thermique, diffusivité thermique, masse volumique, vitesse d'écoulement, débit massique, vitesse du son, constante diélectrique, viscosité, absorption dans l'infrarouge, pression ou température.

**14.** Dispositif de mesure pour déterminer des propriétés de gaz avec un ou plusieurs capteurs (3, 4, 5, 6) pour l'acquisition de grandeurs de mesure physiques ($\mu_j$ *(j = 1,...,m)*) et avec une unité d'analyse (9), **caractérisé en ce que** l'unité d'analyse est conçue pour exécuter un procédé selon l'une des revendications 1 à 13.

**15.** Dispositif de mesure selon la revendication 14, dans lequel l'unité d'analyse forme avec le ou les capteurs une unité de construction, ou dans lequel l'unité d'analyse est réalisée dans une unité de calculateur séparée.

**Fig. 1a**

**Fig. 1b**

**Fig. 2a**

**Fig. 2 b**

Fig. 3

**Fig. 4**

**Fig. 5**

**Fig. 6**

Fig. 7

**Fig. 8**

Fig. 9

Fig. 10

**Fig. 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2015056 A1
- EP 0715169 A1
- DE 19921167 A1
- EP 0939317 A2
- EP 2806271 A1